# EUROPEAN PATENT SPECIFICATION

(11) **EP 3 185 911 B1**
(45) Date of publication and mention of the grant of the patent: **30.12.2020**
(21) Application number: 15748207.6
(22) Date of filing: 28.07.2015
(51) Int. Cl.: A61K 51/04, A61K 103/00

(54) **KIT FOR RADIOLABELLING WITH 68GA COMPRISING A METAL INHIBITOR**
KIT ZUR RADIOMARKIERUNG MIT 68GA MIT EINEM METALLINHIBITOR
KIT POUR LE RADIOMARQUAGE AU 68GA COMPRENANT UN INHIBITEUR MÉTALLIQUE

(30) Priority: 29.08.2014 BE 201400653
(43) Date of publication of application: 05.07.2017
(73) Proprietor: Anmi S.A., 4000 Liège (BE)
(72) Inventor: WOUTERS, Ludovic, 4650 Herve (BE); KAISIN, Geoffroy, 4100 Seraing (BE); LUXEN, André, 4560 Ocquier - Clavier (BE); LÉONARD, Marc, 4400 Flémalle (BE); VOCCIA, Samuel, 4000 Liège (BE)
(74) Representative: De Clercq & Partners
(86) International application number: PCT/EP2015/067211
(87) International publication number: WO 2016/030103

(56) References cited:
- WO-A2-03/059397
- WO-A2-2013/024013
- US-A1- 2013 310 537
- MAUS S. ET AL: "Aspects on radiolabeling of 177Lu-DOTA-TATE: After C18 purification re-addition of ascorbic acid is required to maintain radiochemical purity", INTERNATIONAL JOURNAL OF DIAGNOSTIC IMAGING, vol. 1, no. 1, 21 February 2014 (2014-02-21), pages 5-12, XP002738789, DOI: 10.5430/ijdi.v1n1p5
- HAUBNER ROLAND ET AL: "Development of68Ga-labelled DTPA galactosyl human serum albumin for liver function imaging", EUROPEAN JOURNAL OF NUCLEAR MEDICINE AND MOLECULAR IMAGING, SPRINGER VERLAG, HEIDELBERG, DE, vol. 40, no. 8, 12 April 2013 (2013-04-12) , pages 1245-1255, XP035341691, ISSN: 1619-7070, DOI: 10.1007/S00259-013-2397-8 [retrieved on 2013-04-12]
- PRINSEN K ET AL: "Development and evaluation of a 68Ga labeled pamoic acid derivative for in vivo visualization of necrosis using positron emission tomography", BIOORGANIC & MEDICINAL CHEMISTRY, PERGAMON, GB, vol. 18, no. 14, 15 July 2010 (2010-07-15) , pages 5274-5281, XP027263491, ISSN: 0968-0896 [retrieved on 2010-05-24]
- Bernd Koop ET AL: "Labelling of a monoclonal antibody with 68Ga using three DTPA-based bifunctional ligands and their in vitro evaluation for application in radioimmunotherapy", Radiochimica Acta, vol. 95, no. 1, 1 January 2007 (2007-01-01), pages 39-42, XP55520853, DE ISSN: 0033-8230, DOI: 10.1524/ract.2007.95.1.39
- JAE YEON CHOI ET AL: "Development ofGa-labeled mannosylated human serum albumin (MSA) as a lymph node imaging agent for positron emission tomography", NUCLEAR MEDICINE AND BIOLOGY, ELSEVIER, NY, US, vol. 38, no. 3, 29 September 2010 (2010-09-29), pages 371-379, XP028190375, ISSN: 0969-8051, DOI: 10.1016/J.NUCMEDBIO.2010.09.010 [retrieved on 2010-10-08]
- NEUMANN ET AL: "Native albumin for targeted drug delivery", EXPERT OPINION ON DRUG DELIVERY, INFORMA HEALTHCARE, GB, vol. 7, no. 8, 1 August 2010 (2010-08-01), pages 915-925, XP008170197, ISSN: 1742-5247, DOI: 10.1517/17425247.2010.498474
- LI XIANG ET AL: "Targeting p-selectin by gallium-68-labeled fucoidan positron emission tomography for noninvasive characterization of vulnerable plaques: Correlation with in vivo 17.6t mri", ARTERIOSCLEROSIS, THROMBOSIS, AND VASCULAR BIOLOGY AUGUST 201 LIPPINCOTT WILLIAMS AND WILKINS USA, vol. 34, no. 8, August 2014 (2014-08), pages 1661-1667, ISSN: 1079-5642
- LI XIANG ET AL: "Supplemental Materials", ARTERIOSCLEROSIS, THROMBOSIS, AND VASCULAR BIOLOGY AUG 2014, vol. 34, no. 8, August 2014 (2014-08), pages 1661-1667, ISSN: 1524-4636

## Description

### TECHNICAL FIELD

The present invention is related to kit for radiolabelling

### BACKGROUND

Recently, some very interesting clinical results based on gallium-68 radiolabeled molecules for imaging in vivo by PET were published and presented. These radiotracers are generally made by assembly of a chelating agent with a targeting agent, generally DOTA-functionalized targeting agents, allowing, respectively, the reaction with a metallic radioisotope or radiometal and biological/metabolic activity of the radiotracer. However, due to the short half-life of gallium-68 (68 minutes), the radiotracer, i.e. radiolabelled chelate-functionalized targeting agent, based on this radioisotope are not suitable for long-distance distribution and require on the spot production and suitable production equipment, such as automated synthesizers, for the radiolabelling process, making it difficult for widespread use in routine nuclear medicine.

The labelling reaction with the gallium-68 is performed by chelating the radioactive metal with a suitable chelating agent in a suitable reaction medium, usually in a buffered medium in order to ensure an optimum pH for both the chelation reaction and the gallium solubility.

Gallium-68 itself is obtained from a generator. Said generator is an alternative to the in situ production using a cyclotron or daily delivery of radioisotopes. The system was initially developed for technecium-99. The principle is based on the radiochemical separation between a parent element of long half-life (or nonradioactive elements such as germanium-68) contained in the generator and a daughter element which is a short half-life element resulting from the disintegration of the parent element. The daughter is recovered with excellent radiochemical purity and radionuclidic properties (i.e. without contamination from other radionuclides or other radiochemical impurities) and with good chemical purity (low metal ion content). This separation is made possible by the different chemical properties of the two elements (parent and daughter).

The characteristics of a germanium-68/gallium-68 generator can be summarized as follows:
- The eluate is obtained in an acid solution (0.05M - 5M HCI, specified by the manufacturer of the generator)
- The eluate contains zinc-68, resulting from both the manufacturing process of germanium-68 and disintegration of gallium-68, whose concentration increases continuously in function of time elapsed since the last elution of the generator. Indeed, this zinc-68 accumulates in the generator. This can be detrimental to the performance of radiolabelling since this zinc-68 enters in direct competition with gallium-68 for chelation reactions used for radiolabelling.

- The eluate further contains germanium-68 (the "breakthrough") released from the generator.
- The eluate also contains a variety of metal leaching from the solid phase of the generator column, tubings, but also brought by the HCI used for elution:
   - Microg/ml level: Fe (III), Zn (II), Al (III)
   - Picog/L level: Mn (II), Pb (II), Ti (IV), Cr (III), Ni (II) (Sn (IV))

The efficiency of the chelation reaction is dependent on a suitable pH, but also on possible competition of the metallic impurities mentioned above with the gallium-68 during the chelation reaction as well. In addition, it is generally accepted that heat facilitates the chelation reaction for the most commonly used gallium-68 based radiotracers.

In the state of the art, the presence of metal ions that compete with gallium-68 is generally reduced by pre-labelling purification or fractionation of the eluate (as described in WO 2010/092114). These additional steps however represent a loss of radioactivity resulting from, either wasted time or the process itself. These losses can reach up to 30% of the total radioactivity, respectively, 10% due to decay and 20% coming from the pre-purification process itself.

The possibility of partial chelation of gallium-68 requires, in general, a final post-labelling purification in order to obtain a radiotracer having a radiochemical purity that meets the pharmaceutical specifications (> 90% radiochemical purity). These steps also represent an additional loss of activity that can rise to up to 10% resulting from wasted time or the process itself.

According to known processes, at the end of the radiolabelling, a sequestering agent having a particular affinity for the gallium-68 may be added to chelate the non-reacted part of the isotope. This complex formed by the sequestering agent and the non-reacted gallium-68 is then discarded in order to reach a better radiochemical purity after radiolabelling.

In addition, the need for these pre- and post-labelling purification steps makes these gallium-68 labeled radiotracer synthesis dependent, to some extent, on automation and on the use of a synthesis module. In addition to technical expertise, this requires extra time loss unfavorable to the overall performance.

Due to the short half-life of the radionuclide (68 minutes) and to limited activity supplied by the generator (max. 100mCi), any improvement in order to achieve rapid, direct and high efficiency chelation of target molecules is thus highly desirable.

In order to maintain the pH of the labelling solution in a range where it is possible to ensure both the chelating reaction and the gallium-68 solubility, a buffering medium is generally used. The desired buffer must be nontoxic, must effectively maintain the pH within a range of 3.0 to 5.0, should not compete with gallium-68 ions and have preferably a low capacity for metal chelation with regard to the capacity of the chelating agent as assembled with the targeting agent. It must also be able to tolerate possible small changes in the volume of generator eluate (and therefore the amount of HCI), i.e. it must be strong enough to maintain the pH within the desired range with 10% changes in the volume of eluate.

Management of competing metal impurities is another challenge. It has been shown in WO2013024013 that adding a co-chelating agent could allow inhibition of competing metal impurities. Indeed, any species that would inhibit metal impurities by avoiding or having limited capacity to interfere negatively on the gallium-68 chelation reaction can act as a trap for these impurities. In other words, this inhibitory effect brings the apparent concentration of competitor metal, i.e. the concentration of metallic impurities yet available for chelation to a level which allows high yields and reproducible radiolabelling. This co-chelating agent is by definition different than the chelating agent assembled with the targeting agent.

US patent application 2013/310537 A1 discloses the synthesis of ⁶⁸Ga-labelled DOTA- and NODAGA-conjugated peptides carried at room temperature in an acetate buffer.

PCT patent application WO03/059397 discloses the synthesis of ⁶⁸Ga-labelled NOTA- and DOTA-conjugated peptides carried at room temperature in an ammonium acetate buffer.

In this context, it is clear that a need exists for an improved process for the preparation of ⁶⁸Ga complex which overcomes one or more of the above mentioned problems. This involves identifying an appropriate medium that maintains the pH within a tolerable range, to handle the metal contamination, which avoids the need to heat for promoting the chelating reaction and allows gallium-68 chelation yields upper 90%.

### SUMMARY OF THE INVENTION

The scope of the present invention is defined in the appending claims. The invention relates to the following aspects:
Aspect 1. A radiolabelling kit comprising:
   - a suitable amount of acetate salt or buffer to balance at least the acidic pH eluate from a gallium-68 generator to a pH value ranging from 3 to 5 when said generator is eluted in the kit;
   - a chelate-functionalized targeting agent, said chelate function being able to chelate gallium-68 in the radiolabelling conditions; and
   - a metal inhibitor, which is a co-chelating agent, capable of inactivating contaminating metals other than gallium-68 without interfering with the chelation between gallium-68 and said chelate-functionalized targeting agent, under the conditions of the labelling reaction, wherein said radiolabelling reaction is carried out at a temperature near or equal to room temperature and at a pH of between 3 and 5,
   wherein said metal inhibitor is selected from the group comprising: DOTA, monosaccharides, and disaccharides,
   wherein said targeting agent is a peptide consisting essentially of 2 to 20 amino acids, and
   wherein said chelate functional group of the targeting agent is selected from the group comprising: NOTA, Tris(hydroxypyridinone) (THP), open-chain chelators such as HBED, DFO, EDTA, 6SS, B6SS, PLED, TAME, and YM103; NTP(PRHP)₃, H₂dedpa, (4,6-MeO₂sal)₂-BAPEN, and citrate,
   wherein said metal inhibitor and said chelate-functionalized targeting agent are not chemically linked.
Aspect 2. The kit according to aspect 1, wherein said acetate salt, chelate-functionalized targeting agent and metal inhibitor are (co-)lyophilized.
Aspect 3. The kit according to aspect 1, wherein said chelate-functionalized targeting agent and metal inhibitor are (co-)lyophilized, and wherein said acetate salt or buffer is present separately and can be added subsequently.
Aspect 4. The kit according to aspect 1, wherein said chelate-functionalized targeting agent and metal inhibitor are (co-)lyophilized, an acetate buffer being added subsequently.
Aspect 5. The kit according to anyone of aspects 1 to 4, wherein the acetate salt or acetate salt buffer comprises a quantity of acetate salt adjusted to the type of the gallium-68 generator used.
Aspect 6. The kit according to anyone of aspects 1 to 4, wherein the acetate salt or acetate salt buffer is present in a fixed concentration to balance the acidic pH eluate from a gallium-68 generator to a pH value ranging from 3 to 5 when said generator is eluted in the kit after addition of HCL to the kit.
Aspect 7. The kit according to anyone of aspects 1 to 6, wherein the chelate functional group of the targeting agent is capable of forming a stable complex with Ga³+.
Aspect 8. The kit according to anyone of aspects 1 to 7, wherein the acetate salt is present in an of amount between 5 mg and 1000 mg, preferably in an amount of between 10 mg and 750 mg, more preferably in an amount of between 20 mg and 500 mg.
Aspect 9. The kit according to anyone of aspects 1 to 8, wherein the metal inhibitor is present in a micromolar quantity, preferably in a nanomolar quantity, more preferably in a quantity of below 500 nanomoles, even more preferably in a quantity of below 100 nanomoles.
Also disclosed in a kit according to anyone of aspects 1 to 12, wherein said metal inhibitor and said functionalised agent are chemically linked, preferably wherein said metal inhibitor and said functionalised agent are chemically linked, through a linker that is unstable in the radiolabelling conditions.
Aspect 10. Use of the kit according to anyone of aspects 1 to 9, for radiolabelling a chelate-functionalized targeting agent with gallium-68 carried out at a temperature near or equal to room temperature.
Aspect 11. A method for radiolabelling a chelate-functionalized targeting agent with gallium-68, comprising the elution of a gallium-68 generator with an eluent comprising an acid, in a kit according to anyone of aspects 1 to 10.
Aspect 12. The method according to aspect 11, wherein the acid is HCI.
Aspect 13. The method according to aspect 11 or 12, comprising additionally the step of adding HCI to the kit before elution.
Aspect 14. The method according to anyone of aspects 11 to 13, wherein the radiolabelling is performed at a pH comprised between 3 and 5, preferably between 3,5 and 4,5, more preferably between 3,9 and 4,3.
Aspect 15. The method according to anyone of aspects 11 to 14, wherein the radiolabelling reaction is carried out at a temperature of below 50°C, preferably of ambient or room temperature (e.g. of between 20 and 30°C).
   Also disclosed is a solution obtainable by elution of a gallium-68 generator with an eluent comprising an acid, in a kit according to anyone of aspects 1 to 9.
   Also disclosed is asolution obtainable by elution of a gallium-68 generator with an eluent comprising a base, in a kit according to anyone of aspects 1 to 9.
   Also disclosed is asolution obtainable by elution of a gallium-68 generator with an eluent that is concentrated or purified prior to its transfer in a kit according to anyone of aspects 1 to 9.
   Preferably said solution has a pH of between 3 and 5, preferably between 3,5 and 4,5, more preferably between 3,9 and 4,3.
Aspect 16. A process for preparing a radiolabelling kit according to anyone of aspects 1 to 9, comprising the steps of:
   a1) preparing or providing a solution comprising suitable amount of acetate salt or buffer to balance at least the acidic pH eluate from a gallium-68 generator to a pH value ranging from 3 to 5,
   a2) preparing or providing a chelate-functionalized targeting agent;
   a3) preparing or providing an inhibitor of metal; and
   b) lyophilizing the solution obtained in steps a1) to a3).
Aspect 17. A process for preparing a radiolabelling kit according to anyone of aspects 1 to 9, comprising the steps of:
   a) preparing or providing a solution comprising a chelate-functionalized targeting agent and an inhibitor of metal;
   b) lyophilizing the solution obtained in step a), and
   c1) adding the acetate salt as a powder in the obtained lyophilized product in step b), or
   c2) adding an acetate buffer in the obtained lyophilized product in step b); or
   comprising the steps of:
   a) preparing a solution comprising the acetate salt, a chelate-functionalized targeting agent and the metal inhibitor; and
   b) optionally freeze the solution obtained in step a)..
Aspect 18. A process for preparing a kit according to anyone of aspects 1 to 9, comprising the steps of:
   a) preparing a solution comprising a chelate-functionalized targeting agent and an inhibitor of metal;
   b) lyophilizing the solution obtained in step a), and
   c) adding an acetate buffer in the obtained lyophilized product in step b).
      Also disclosed is a process for preparing a kit according to anyone of aspects 1 to 9, comprising the steps of:
      a) preparing a solution comprising the acetate salt, a chelate-functionalized targeting agent and an inhibitor of metal; and
      b) optionally freeze the solution obtained in step a).
Aspect 19. A process for radiolabelling a target agent with gallium-68, wherein a metal inhibitor is included either in the eluate of the gallium-68 generator, or in the HCI solution added before elution of the gallium-68 generator, wherein said metal inhibitor is present in the radiolabelling solution, wherein said metal inhibitor is selected from the group comprising: DOTA, monosaccharides, and disaccharides,
   wherein said targeting agent is a peptide consisting essentially of 2 to 20 amino acids, and
   wherein said chelate functional group of the targeting agent is selected from the group comprising: NOTA, Tris(hydroxypyridinone) (THP), open-chain chelators such as HBED, DFO, EDTA, 6SS, B6SS, PLED, TAME, and YM103; NTP(PRHP)₃, H₂dedpa, (4,6-MeO₂sal)₂-BAPEN, and citrate,
   wherein said metal inhibitor and said chelate-functionalized targeting agent are not chemically linked.

### DETAILED DESCRIPTION

As used herein, the singular forms "a", "an", and "the" include both singular and plural referents unless the context clearly dictates otherwise.
The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of".

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of and from the specified value, in particular variations of +/-10% or less, preferably +/-5% or less, more preferably +/-1% or less, and still more preferably +/-0.1 % or less of and from the specified value, insofar such variations are appropriate to perform in the disclosed methods, uses and kits. It is to be understood that the value to which the modifier "about" refers is itself also specifically, and preferably, disclosed.

Whereas the term "one or more", such as one or more members of a group of members, is clear per se, by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Unless otherwise specified, all terms used herein, including technical and scientific terms, have the meaning as commonly understood by one of ordinary skill in the art to which this disclosure belongs. By means of further guidance, term definitions may be included to better appreciate the teaching of the present disclosure.

In the following passages, different aspects are defined in more detail. Every aspect so defined may be combined with each of the other aspects unless stated otherwise. In particular, any feature indicated as being preferred or advantageous in one aspect may be combined with any other aspect or aspects indicated as being preferred or advantageous.

The present invention overcomes one or more of the problems identified and observed in the state of the art and allows the direct radiolabelling of a chelate-functionalized targeting agent with gallium-68 at a temperature below 50°C and preferably at room temperature, using a kit as described herein, this gallium-68 being eluted from a germanium-68/gallium-68 generator in an acidic aqueous solution.

Accordingly, in one aspect, the disclosure provides a kit comprising:
- A suitable amount of acetate salt to balance at least the acidic pH eluate from a gallium-68 generator to a pH value ranging from 3 to 5 when said generator is eluted in the kit; and
- A chelate-functionalized targeting agent, able to chelate gallium-68 in the radiolabeling conditions
- A metal inhibitor, which is a co-chelating agent, capable of inactivating metals other than gallium-68 without interfering with the chelation between gallium-68 and the said chelate-functionalized targeting agent, under the conditions of the labelling reaction. In other words, said metal inhibitor is selected for its ability to chelate contaminating metals interfering and competing with the chelation of gallium-68 while being mostly unable gallium-68 in the said conditions of the labelling reaction as opposed to the chelate-functionalized targeting agent.

Said kit being suitable to perform the radiolabelling reaction of said chelate-functionalized targeting agent with gallium-68 as carried out at a temperature near or equal to room temperature, preferably at a temperature below 50°C and more preferably at room temperature.

The disclosure also relates to a kit wherein the acetate salt, the chelate-functionalized targeting agent and the metal inhibitor are (co-) lyophilized.

The disclosure also relates to a kit wherein the chelate-functionalized targeting agent and the metal inhibitor are (co-)lyophilized, the acetate salt being added subsequently.

The disclosure also relates to a kit wherein the chelate-functionalized targeting agent and the metal inhibitor are (co-)lyophilized, an acetate buffer being added subsequently.

The disclosure also relates to a kit wherein the acetate salt, the chelate-functionalized targeting agent and the metal inhibitor are solubilized and further frozen.

The kit as described herein can not only provide an optimum pH for carrying out the chelation reaction or radiolabelling, but also allows to tolerate or manage the variation of the eluate volume and acidity associated with different types of gallium-68 generators, through the use of a suitable amount of acetate salt that when mixed with the acid generator eluate, form an acetic acid/acetate buffer having an acid pH comprised in the interval 3-5. In these conditions, the amount of non-chelated gallium-68 because of a too low or too high pH, which leads respectively to a high content of free gallium-68 cations or to gallium-68 hydroxides (gallium colloids), is minimized.

In addition, the acetate buffer is well tolerated as a buffer or as an excipient for pharmaceuticals.

Furthermore, the present inventors have found that a metal inhibitor can be used in the radiolabelling method for neutralizing, at least partially, interfering species and allows the gallium-68 to react with the chelate-functionalized targeting agent. These metal inhibitors may temporarily or permanently remove metals that compete with gallium-68 for the reaction with the chelate-functionalized targeting agent. Said metal inhibitor is thus unable to chelate gallium-68 in the said conditions of the labelling reaction, but chelate other metals interfering with the chelation of gallium-68 by the chelate-functionalized targeting agent. The presence of a metal inhibitor during the radiolabelling reaction provides an advantageous alternative to current approaches for managing the presence of metallic impurities, such as increasing the amount chelate-functionalized targeting agent, or the pre- treatment of the eluate of the generator, since these additional purification steps consume time (and radioactivity).

These aspects as described herein advantageously allow obtaining an appropriate chelation yield, particularly of about 90 % and more, and therefore a sufficient radiochemical purity without any preliminary or further final purification.

The presence of a chelate-functionalized targeting agent, an acetate salt and a metal inhibitor in the kit advantageously allows to directly elute gallium-68 generator in the kit and performing the radiolabelling reaction without the need for any prior or subsequent operation.

In addition, all kit components as described herein can be lyophilized altogether or frozen which ensures a longer shelf life.

Thus, the main advantages of a kit as disclosed herein that differentiate said kit from the state of the art are:
- A completely dry or frozen kit that allows a better shelf life of the chelate-functionalized targeting agent;
- The possibility of radiolabelling without the need for an automated synthesizer;
- The possibility of a radiolabelling without the need for heating;
- The presence of a metal inhibitor which advantageously allows to use less chelate-functionalized targeting agent and allowing the implementation of more affordable radiopharmaceutical synthesis;
- The presence of a metal inhibitor which advantageously allows to improve the radiolabelling yields;
- The fact that any brand generator can be used with this kit provided as acetate or partially neutralized with HCI so that when mixed with the acid generator eluate, the optimal pH for the radiolabelling is obtained.

As used herein, "acetate" refers to the anionic molecule CH₃COO-. The term "acetate salt" herein is meant any metal salt acetate. Non-limiting examples of acetate salts include sodium acetate, potassium acetate, aluminium acetate, and ammonium acetate. Preferably sodium acetate is used in the kits as described herein. Said acetate salt can be present in solid form or can be comprised in a buffered solution or buffer.

The amount of salt of the acetate present in the kit as described herein can be adapted according to the type and / or the kind of gallium-68 generator, in particular the quantity of acetate salt present in the kit is able to balance the pH, i.e. to manage the quantity of HCI as eluted from a gallium-68 generator such that the resulting solution has a pH between 3 and 5, preferably between 3.5 and 4.7, preferably between 3.9 and 4.5.

Alternatively, the kit as described herein may comprise a fixed quantity of acetate salt. The amount of HCI differences from the generator eluate (depending on the type and / or the generator brand gallium-68) can then be adjusted by adding an appropriate amount of HCI to the kit as described herein prior to elution. The amount of HCI added to the kit as described herein is partially neutralizing the acetate salt such that the non-neutralized acetate salt is able to balance the pH of a quantity of HCI from a generator eluate such that the resulting solution has a pH between 3 and 5, preferably between 3.5 and 4.7, preferably between 3.9 and 4.5.

Preferably, the acetate salt is present in the kit as taught herein in an amount between about 1 mg and about 1000 mg, preferably in an amount between about 10 mg and about 750 mg, more preferably in an amount between about 20 mg and about 500 mg.

Metal inhibitors used in the present disclosure are selected for their ability to inhibit the competing metals, without (substantially) inhibiting gallium-68 ions in their chelation reaction with the chelate-functionalized targeting agent. Indeed, these metal inhibitors should (substantially) not interfere negatively on the main radiolabelling reaction or lead to the formation of secondary radiolabeled species. In other words metal inhibitors should have a limited or no capacity to complex gallium-68 in the conditions used for the radiolabelling reaction, i.e. below 50°C in an acetate buffer between pH 3 and pH 5. Limited means at least 100 times less than the chelating agent used for the chelate-functionalized targeting agent.

It is interesting to note that the function of metal inhibitors in the present disclosure is the opposite of the function of the sequestering agents used in the prior art. Indeed, according to known methods, at the end of the labelling reaction, a sequestering agent having a particular affinity for the gallium-68 may be added to chelate the unreacted portion of the isotope, whereas, according to the present disclosure an agent capable of reducing the competition of metallic impurities other than the gallium-68 is added at the beginning of the reaction.

In addition, being able to perform the radiolabelling reaction at a temperature close to room temperature (<50°C) advantageously allows the use of metal inhibitors that would not be usable at the usual temperatures of radiolabelling DOTA-functionalized targeting agents by such as used in WO2013024013, because they would be entering in direct competition with gallium-68 at such temperatures of above 50°C. The temperature is therefore also described herein as a parameter for adjusting the reactivity of the metal inhibitor.

Metal inhibitors for use with the kit of the invention are selected from the group comprising: DOTA, monosaccharides and disaccharides.

As disclosed herein, a "metal inhibitor" refers to any molecule capable of interacting with, or competing metals, or the chelating moiety of the chelate-functionalized targeting agent or with gallium-68 directly, to inhibit wholly or partially the chelation the chelate-functionalized targeting agent said competing metals and/or promote the chelating of gallium-68 by said targeting agent. Such metal inhibitors should have a limited or no capacity to complex gallium-68 in the conditions used for the radiolabelling reaction, i.e. below 50°C in an acetate buffer between pH 3 and pH 5. Limited means at least 100 times less than the chelating agent used for the chelate-functionalized targeting agent.

Examples of metal inhibitors disclosed herein are: DOTA and its derivatives, such as, DOTATOC, DOTANOC, DOTATA, TRITA, DO3A-Nprop, BisDO3A and TrisDO3A; DTPA and its derivatives such as tetra-tBu-DTPA, p-SCN-Bz-DTPA, MX-DTPA and CHX-DTPA; and sugars. Sugars can be monosaccharides or derivatives of monosaccharides such as tetracetose, pentacetose, hexacétose, tetrose, pentose, hexose, D-mannose, D-fructose, and derivatives; and / or disaccharides and their derivatives such as maltose and its derivatives; and / or polysaccharides and their derivatives such as dextrins, cyclodextrins, cellulose and derivatives thereof.

Preferably, the metal inhibitor is present in the kit as described herein in micromolar amounts, preferably in nanomolar quantities, preferably in an amount of less than 500 nanomolar, still more preferably in an amount less than 100 nanomoles.

It is important to note that metal inhibitors as shown above can also be advantageously used in chelation reactions wherein other buffers than buffered acetic acid / acetate are used.

Metal inhibitors as shown above can also be advantageously used in chelation reactions wherein said metal inhibitor is included in the eluent generator, in the HCI solution, or in water possibly added before elution of the generator. Said metal inhibitor is thus found in the radiolabelling solution. Also disclosed is the situation wherein the metal inhibitor is chemically bound to the chelate-functionalized targeting agent. This chemical bond can or cannot be a labile bond under the conditions of radiolabelling with the chelate-functionalized targeting agent. This means that in the conditions of radiolabelling the metal inhibitor is formed and released in situ.

As used herein, a "chelate-functionalized targeting agent" refers to a targeting agent capable of being labeled with a radioisotope such as for example gallium-68, by means of a chelating agent which is bound to the targeting molecule.

The chelating agents for use with the present invention are selected from the group comprising: NOTA, Tris(hydroxypyridinone) (THP), open-chain chelators such as HBED, DFO, EDTA, 6SS, B6SS, PLED, TAME, and YM103; NTP(PRHP)₃, H₂dedpa, (4,6-MeO₂sal)₂-BAPEN, and citrate.

Chelating agents disclosed herein are those which form stable chelates with Ga3+, in particular 68-Ga3+ (the radioisotope generator eluted from a germanium-68/gallium-68 generator using HCI), at least for a time sufficient for diagnostic investigations using such radiolabelled targeting agents. Suitable chelating agents include aliphatic amines, linear or macrocyclic such as macrocyclic amines with tertiary amines. Examples of suitable chelating agents include: NOTA and its derivatives, such as TACN, TACN-TM, DTAC, H3NOKA, NODASA, NODAGA, NOTP, NOTPME, PrP9, TRAP, NOPO, TETA; Tris(hydroxypyridinone) (THP) and derivatives, chelates open chain such as HBED, DFO or desferrioxamine or desferal, EDTA, 6SS, B6SS, PLED, TAME, YM103; NTP(PRHP)₃; the H₂dedpa and its derivatives such as H₂dp-bb-NCS, and H₂dp-N-NCS; (4,6-MeO₂sal)2-BAPEN; and citrate.

The targeting agent that is chelate-functionalized for use in the current invention is a peptide consisting essentially of 2 to 20 amino acids.

A targeting agent as disclosed herein can be a peptide, a polypeptide, a protein, a vitamin, a saccharide, for example a monosaccharide or a polysaccharide, an antibody and its derivatives such as nanobodies, diabodies, antibodies fragments, nucleic acid, an aptamer, an antisense oligonucleotide, an organic molecule, or any other biomolecule that is able to bind to a certain diagnostic target or to express a certain metabolic activity.

Chelate-functionalized targeting agents as described herein preferably have a capacity of biological targeting. Examples of suitable targeting agents include molecules that target VEGF receptors, analogs of bombesin or GRP receptor targeting molecules, molecules targeting somatostatin receptors, RGD peptides or molecules targeting αvβ3 and αvβ5, annexin V or molecules targeting the apoptotic process, molecules targeting estrogen receptors, biomolecules targeting the plaque. More generally, a list targeting molecules, organic or not, functionalized by a chelating agent can be found in the journal of Velikyan et al., Theranostic 2014, Vol. 4, Issue 1 "Prospective of 68Ga-Radiopharmaceutical Development."

In some embodiments, the metal inhibitor is included in the eluent generator, in the HCI solution, or possibly in the added water prior to elution of the generator. Said metal inhibitor and is thus found in the radiolabelling solution.

The various components of the kit as described herein are preferably present in a container or vial, preferably a siliconized glass vial. However, also a kit wherein the individual components are present in separate containers or vials is envisaged.

The disclosure further provides a method for radiolabelling a targeting agent with gallium-68, said method comprising the elution of a gallium-68 generator with an eluent comprising an acid, in a kit as described herein, e.g. comprising the metal inhibitor, the chelate-functionalized targeting agent and acetate salt.

As indicated above, when the chelate-functionalized targeting agent is included in the kit, a gallium-68 generator can be eluted directly into the kit. In other embodiments, the chelate-functionalized targeting agent can be added to a kit comprising the acetate salt and a metal-inhibiting agent as described herein, prior to elution.

In some embodiments, the gallium-68 generator is eluted directly into the kit. In other embodiments, water is added to the solution prior to elution.

In some embodiments of the present disclosure, an appropriate amount of HCI is added to the solution prior to elution. Said HCI is added to partially neutralize the acetate. The amount of HCI added, preferably partially neutralizes the quantity of acetate salt in such a manner that the remaining quantity of acetate salt, i.e. unneutralized acetate salt, is able to balance the pH of said amount of HCI from the generator eluate (and thus dedicated to one type or brand of given generator) such that the pH of the solution obtained for the radiolabelling reaction or chelating reaction, resulting from the addition of HCI and the generator eluate in the kit as described herein, is in a pH range between 3 and 5, preferably between 3.5 and 4.5, preferably between 3.9 and 4.3. Said HCI may be added directly to the solution, or after a certain amount of water is added to said kit.

All gallium-68 generator may be used in the methods of the present disclosure. Typically, a commercial gallium-68 generator comprises a column on which the germanium-68 is fixed. A gallium-68 generator is typically eluted with an eluent comprising an acid, preferably HCI. Therefore, in preferred embodiments of the method, as taught herein, the gallium-68 generator is eluted with an eluent comprising HCI.

After elution of the gallium-68 generator in the kit as described herein, the solution obtained is left to react in the radiolabelling reaction for a short period of time, in particular between about 2 minutes and about 60 minutes, preferably from about 2 minutes to about 30 minutes, for example about 10 minutes.

Preferably, the radiolabelling reaction or chelation is performed at a temperature below 50° C, preferably of below 45°C, below 40°C, below 35°C, or below 30°C, most preferably at room temperature, e.g. between 20 and 25°C.

Preferably, the radiolabelling reaction or chelation is performed at a pH between about 3 and about 5, more preferably between about 3.5 and about 4.5, more preferably between about 3.9 and about 4.3.

The disclosure also encompasses the solution obtained by elution of a gallium-68 generator with an eluent comprising an acid, preferably HCI, in a kit as taught herein.

Preferably, said solution has a pH between about 3 and about 5, preferably between about 3.5 and about 4.5, more preferably between about 3.9 and about 4.3.

The disclosure also discloses a gallium-68 radiolabeled targeting agent, obtained by anyone of the methods as described herein.

In one aspect, the disclosure also provides a preparation method of a kit as described herein, said method comprising the steps of:
a) preparing a solution comprising the acetate salt, a chelate-functionalized targeting agent and an inhibitor of metal; and
b) lyophilizing the solution obtained in step a).

Alternatively, the disclosure further provides a process for preparing a kit of the disclosure comprising the steps of:
a) preparing a solution comprising a chelate-functionalized targeting agent and an inhibitor of metal; and
b) lyophilizing the solution obtained in step a).
c) adding the acetate salt as a powder in the obtained lyophilized product in step b).

Further alternatively, the disclosure further provides a process for preparing a kit of the disclosure comprising the steps of:
a) preparing a solution comprising a chelate-functionalized targeting agent and an inhibitor of metal; and
b) lyophilizing the solution obtained in step a).
c) adding an acetate buffer in the obtained lyophilized product in step b).

Finally, the disclosure further provides a process for preparing a kit of the disclosure comprising the steps of:
a) preparing a solution comprising the acetate salt, a chelate-functionalized targeting agent and an inhibitor of metal; and
b) optionally freeze the solution obtained in step a).

The above aspects and embodiments are further supported by the following examples.

### Examples

### Example 1: Generator E & Z / NODAGA peptide without metal inhibitor:

### Labelling a peptide with a 68Ga eluate of 5 mL of 0.1 M HCl

A commercial gallium-68 generator 1850 MBq (Eckert & Ziegler) is eluted with 5 mL of 0.1M HCI (Ultrapure grade) directly into a flask containing 150 mg of sodium acetate (Ultrapure grade) lyophilized, 240 µl of HCI 3M (Ultrapure grade), 760 µl of Milli-Q and 50 µg lyophilized NODAGA-NOC. The flask was left for 10 min at room temperature. The product is obtained with a radiochemical purity of 64% according to TLC analysis of the reaction medium.

### Example 2: Similarly to what was done in Example 1 different combinations were tested and are summarized in the table below:

| **Entry** | **Acetate** | **Chelating agent use in the chelate-functionalized targeting agent** | **Inhibitor** | **Generator** | **Radiolabelling yield for 10 minutes, room T° vs radiolabelling yield without metal inhibitor using similar conditions** | **Generator cleaniliness** | **Kit Preparati on*** |
|---|---|---|---|---|---|---|---|
| 1 | 150mg | NOTA 25µg | DOTA | E&Z | 82% vs 51% | Generator cleaned | A |
| 2 | 150mg | NOTA 25µg | Fructose | E&Z | 87% vs 51% | Generator cleaned | A |
| 3 | 150mg | NOTA 25µg | Betacyclodextrin | E&Z | 83% vs 51% | Generator cleaned | A |
| 4 | 150mg | NODAGA 25µg | Betacyclodextrin | E&Z | 95% vs 64% | Generator cleaned | A |
| 5 | 150mg | HBED 25µg | Betacyclodextrin | E&Z | 91% vs 77% | Generator cleaned | A |
| 6 | 150mg | HBED 25µg | Fructose | E&Z | 94% vs 77% | Generator cleaned | A |
| 7 | 150mg | NOTA 10µg | Fructose | E&Z | 85% vs 39% | Generator cleaned | A |
| 9 | 150mg | NODAGA 10µg | Betacyclodextrin | E&Z | 84% vs 55% | Generator cleaned | A |
| 9 | 150mg | HBED 10µg | Betacyclodextrin | E&Z | 87% vs 51% | Generator cleaned | A |
| 10 | 150mg | NODAGA 50µg | Betacyclodextrin | ITG | 94% vs 46% | Generator cleaned | A |
| 11 | 150mg | NODAGA 50µg | Beta-cyclodextrin | E&Z | 97% vs 70% | Generator cleaned | A |
| 12 | 150mg | NODAGA 50µg | D-Mannose | E&Z | 91% vs 44% | Generator not cleaned | A |
| 13 | 150mg | NODAGA 50µg | DOTA | E&Z | 95% vs 70% | Generator cleaned | A |
| 14 | 150mg | NODAGA 50µg | Beta-cyclodextrin | iThemba | 91% vs 61% | Generator cleaned | A |
| 15 | 150mg | NODAGA 50µg | Fructose | E&Z | 95% vs 70% | Generator cleaned | A |
| 16 | 150mg | HBED 20µg | DOTA | ITG | 91% vs 75% | Generator cleaned | A |
| 17 | 150mg | NODAGA 25µg | D-Mannose | ITG | 95% vs 60% | Generator cleaned | A |
| 18 | 150mg | NODAGA 25µg | Beta-cyclodextrin | ITG | 96% vs 60% | Generator cleaned | A |
| 19 | 150mg | NODAGA 25µg | tetra-tBu-DTPA | ITG | 89% vs 60% | Generator cleaned | A |
| 20 | 150mg | NODAGA 25µg | Beta-cyclodextrin | ITG | 96% vs 61% | Generator cleaned | B |
| 21 | 150mg | NODAGA 25µg | DOTA | E&Z | 94% vs 64% | Generator cleaned | B |
| 22 | 150mg | NODAGA 25µg | DOTA | E&Z | 89% vs 64% | Generator cleaned | C |
| 23 | 150mg | NODAGA 25µg | Glucose | ITG | 89% vs 61% | Generator cleaned | C |
| 24 | 150mg | DFO 10µg | DOTA | ITG | 98% vs 85% | Generator cleaned | A |

| | | | | | | | |
|---|---|---|---|---|---|---|---|
| * | | | | | | | |

A= a preparation method comprising the steps of:
   a) preparing a solution comprising the acetate salt, a chelate-functionalized targeting agent and an inhibitor of metal; and
   b) lyophilizing the solution obtained in step a).
B=a preparation method comprising the steps of:
   a) preparing a solution comprising a chelate-functionalized targeting agent and an inhibitor of metal; and
   b) lyophilizing the solution obtained in step a).
   c) adding the acetate salt as a solid
C=a preparation method comprising the steps of:
   a) preparing a solution comprising a chelate-functionalized targeting agent and an inhibitor of metal; and
   b) lyophilizing the solution obtained in step a).
   c) adding the acetate salt as a buffer solution adapted to the generator used

To conclude, the results above clearly show the increased gallium-68 radiolabelling yield of about 90% or more in all set-ups where a metal inhibitor as defined herein is used in addition to the chelator-functionalized targeting agent. If said agent is not added, much lower yields are obtained. The yield is virtually independent of the use of acetate in solid form or in buffer form. Also when the acetate salt is co-lyophilized with the metal inhibitor and the chelator-functionalized targeting agent, a very good yield is obtained.

## Claims

1. A radiolabelling kit comprising:
- a suitable amount of acetate salt or buffer to balance at least the acidic pH eluate from a gallium-68 generator to a pH value ranging from 3 to 5 when said generator is eluted in the kit;
- a chelate-functionalized targeting agent, said chelate function being able to chelate gallium-68 in the radiolabelling conditions; and
- a metal inhibitor, which is a co-chelating agent, capable of inactivating contaminating metals without interfering with the chelation between gallium-68 and said chelate-functionalized targeting agent, under the conditions of the labelling reaction,
wherein said radiolabelling reaction is carried out at a temperature near or equal to room temperature and at a pH of between 3 and 5,
wherein said metal inhibitor is selected from the group comprising: DOTA, monosaccharides, and disaccharides,
wherein said targeting agent is a peptide consisting essentially of 2 to 20 amino acids, and
wherein said chelate functional group of the targeting agent is selected from the group comprising: NOTA, Tris(hydroxypyridinone) (THP), open-chain chelators such as HBED, DFO, EDTA, 6SS, B6SS, PLED, TAME, and YM103; NTP(PRHP)₃, H₂dedpa, (4,6-MeO₂sal)₂-BAPEN, and citrate,
wherein said metal inhibitor and said chelate-functionalized targeting agent are not chemically linked.

2. The kit according to claim 1, wherein said acetate salt, chelate-functionalized targeting agent and metal inhibitor are (co-)lyophilized.

3. The kit according to claim 1, wherein said chelate-functionalized targeting agent and metal inhibitor are (co-)lyophilized, and wherein said acetate salt or buffer is present separately and can be added subsequently, or
wherein said chelate-functionalized targeting agent and metal inhibitor are (co-)lyophilized, and acetate buffer being added subsequently.

4. The kit according to anyone of claims 1 to 3, wherein the acetate salt or acetate salt buffer comprises a quantity of acetate salt adjusted to the type of the gallium-68 generator used.

5. The kit according to anyone of claims 1 to 3, wherein the acetate salt or acetate salt buffer is present in a fixed concentration to balance the acidic pH eluate from a gallium-68 generator to a pH value ranging from 3 to 5 when said generator is eluted in the kit after addition of HCI to the kit.

6. The kit according to anyone of claims 1 to 5, wherein the chelate functional group of the targeting agent is capable of forming a stable complex with Ga³⁺.

7. The kit according to anyone of claims 1 to 6, wherein the acetate salt is present in an of amount between 5 mg and 1000 mg, preferably in an amount of between 10 mg and 750 mg, more preferably in an amount of between 20 mg and 500 mg.

8. The kit according to anyone of claims 1 to 7, wherein the metal inhibitor is present in a micromolar quantity, preferably in a nanomolar quantity, more preferably in a quantity of below 500 nanomoles, even more preferably in a quantity of below 100 nanomoles.

9. Use of the kit according to anyone of claims 1 to 8, for radiolabelling a chelate-functionalized targeting agent with gallium-68 carried out at a temperature near or equal to room temperature.

10. A method for radiolabelling a chelate-functionalized targeting agent with gallium-68, comprising the elution of a gallium-68 generator with an eluent comprising an acid, in a kit according to anyone of claims 1 to 8, preferably wherein the acid is HCI.

11. The method according to claim 10, comprising additionally the step of adding HCI to the kit before elution.

12. The method according to claim 10 or 11, wherein the radiolabelling is performed at a pH comprised between 3 and 5, preferably between 3,5 and 4,5, more preferably between 3,9 and 4,3.

13. The method according to anyone of claims 10 to 12, wherein the radiolabeling reaction is carried out at a temperature of below 50°C, preferably of ambient or room temperature (e.g. of between 20 and 30°C).

14. A process for preparing a radiolabelling kit according to anyone of claims 1 to 8, comprising the steps of:
a1) preparing or providing a solution comprising suitable amount of acetate salt or buffer to balance at least the acidic pH eluate from a gallium-68 generator to a pH value ranging from 3 to 5,
a2) preparing or providing a chelate-functionalized targeting agent and
a3) preparing or providing the metal inhibitor; and
b) lyophilizing the solution obtained in steps a1) to a3).

15. A process for preparing a radiolabelling kit according to anyone of claims 1 to 8, comprising the steps of:
a) preparing or providing a solution comprising a chelate-functionalized targeting agent and the metal inhibitor;
b) lyophilizing the solution obtained in step a), and
c1) adding the acetate salt as a powder in the obtained lyophilized product in step b), or
c2) adding an acetate buffer in the obtained lyophilized product in step b); or
comprising the steps of:
a) preparing a solution comprising the acetate salt, a chelate-functionalized targeting agent and the metal inhibitor; and
b) optionally freeze the solution obtained in step a).

16. A process for radiolabelling a chelate-functionalized targeting agent with gallium-68, wherein a metal inhibitor is included either in the eluate of the gallium-68 generator, or in the HCI solution added before elution of the gallium-68 generator, wherein said metal inhibitor is present in the radiolabelling solution,
wherein said metal inhibitor is selected from the group comprising: DOTA, monosaccharides, and disaccharides,
wherein said targeting agent is a peptide consisting essentially of 2 to 20 amino acids, and
wherein said chelate functional group of the targeting agent is selected from the group comprising: NOTA, Tris(hydroxypyridinone) (THP), open-chain chelators such as HBED, DFO, EDTA, 6SS, B6SS, PLED, TAME, and YM103; NTP(PRHP)₃, H₂dedpa, (4,6-MeO₂sal)₂-BAPEN, and citrate,
wherein said metal inhibitor and said chelate-functionalized targeting agent are not chemically linked.

## Patentansprüche

1. Kit zur radioaktiven Markierung, umfassend:
- eine geeignete Menge Acetatsalz oder -puffer zum Ausgleichen wenigstens des Saurer-pH-Eluats aus einem Gallium-68-Generator auf einen pH-Wert im Bereich von 3 bis 5 beim Eluieren des Generators im Kit;
- ein mit einer Chelatfunktion versehenes Targeting-Mittel, wobei die Chelatfunktion bei den Bedingungen der radioaktiven Markierung ein Gallium-68-Chelat bilden kann; und
- einen Metallhemmer, bei dem es sich um einen Co-Chelatbildner handelt, der kontaminierende Metalle unter den Bedingungen der Markierungsreaktion inaktivieren kann, ohne die Chelatbildung zwischen Gallium-68 und dem mit einer Chelatfunktion versehenen Targeting-Mittel zu stören,
wobei die Reaktion zur radioaktiven Markierung bei einer Temperatur nahe oder gleich Raumtemperatur und bei einem pH zwischen 3 und 5 durchgeführt wird, wobei der Metallhemmer ausgewählt ist aus der Gruppe umfassend: DOTA, Monosaccharide und Disaccharide, wobei es sich bei dem Targeting-Mittel um ein Peptid handelt, das im Wesentlichen aus 2 bis 20 Aminosäuren besteht, und
wobei die Chelatfunktionsgruppe des Targeting-Mittels ausgewählt ist aus der Gruppe umfassend: NOTA, Tris(hydroxypyridinon) (THP), offenkettige Chelatbildner wie HBED, DFO, EDTA, 6SS, B6SS, PLED, TAME und YM103; NTP(PRHP)₃, H₂dedpa, (4,6-MeO₂sal)₂-BAPEN und Citrat,
wobei der Metallhemmer und das mit einer Chelatfunktion versehene Targeting-Mittel nicht chemisch verknüpft sind.

2. Kit nach Anspruch 1, wobei das Acetatsalz, das mit einer Chelatfunktion versehene Targeting-Mittel und der Metallhemmer (co-)lyophilisiert sind.

3. Kit nach Anspruch 1, wobei das mit einer Chelatfunktion versehene Targeting-Mittel und der Metallhemmer (co-)lyophilisiert sind und wobei das Acetatsalz bzw. der Acetatpuffer getrennt vorliegt und anschließend zugegeben werden kann, oder
wobei das mit einer Chelatfunktion versehene Targeting-Mittel und der Metallhemmer (co-)lyophilisiert sind und der Acetatpuffer anschließend zugegeben wird.

4. Kit nach einem der Ansprüche 1 bis 3, wobei das Acetatsalz bzw. der Acetatsalzpuffer eine Acetatsalzmenge umfasst, die an den Typ des verwendeten Gallium-68-Generators angepasst ist.

5. Kit nach einem der Ansprüche 1 bis 3, wobei das Acetatsalz bzw. der Acetatsalzpuffer in einer festgelegten Konzentration zum Ausgleichen des Saurer-pH-Eluats aus einem Gallium-68-Generator auf einen pH-Wert im Bereich von 3 bis 5 beim Eluieren des Generators im Kit nach Zugabe von HCl zum Kit vorliegt.

6. Kit nach einem der Ansprüche 1 bis 5, wobei die Chelatfunktionsgruppe des Targeting-Mittels einen stabilen Komplex mit Ga³⁺ bilden kann.

7. Kit nach einem der Ansprüche 1 bis 6, wobei das Acetatsalz in einer Menge zwischen 5 mg und 1000 mg, bevorzugt in einer Menge zwischen 10 mg und 750 mg, stärker bevorzugt in einer Menge zwischen 20 mg und 500 mg vorliegt.

8. Kit nach einem der Ansprüche 1 bis 7, wobei der Metallhemmer in einer mikromolaren Menge, bevorzugt in einer nanomolaren Menge, stärker bevorzugt in einer Menge unterhalb 500 Nanomol, noch stärker bevorzugt in einer Menge unterhalb 100 Nanomol vorliegt.

9. Verwendung des Kits nach einem der Ansprüche 1 bis 8, zur radioaktiven Markierung eines mit einer Chelatfunktion versehenen Targeting-Mittels mit Gallium-68, durchgeführt bei einer Temperatur nahe oder gleich Raumtemperatur.

10. Methode zur radioaktiven Markierung eines mit einer Chelatfunktion versehenen Targeting-Mittels mit Gallium-68, umfassend die Elution eines Gallium-68-Generators mit einem eine Säure umfassenden Elutionsmittel, in einem Kit nach einem der Ansprüche 1 bis 8, vorzugsweise wobei es sich bei der Säure um HCl handelt.

11. Methode nach Anspruch 10, umfassend zusätzlich den Schritt der Zugabe von HCl zum Kit vor der Elution.

12. Methode nach Anspruch 10 oder 11, wobei die radioaktive Markierung bei einem zwischen 3 und 5, bevorzugt zwischen 3,5 und 4,5, stärker bevorzugt zwischen 3,9 und 4,3 liegenden pH-Wert erfolgt.

13. Methode nach einem der Ansprüche 10 bis 12, wobei die Reaktion zur radioaktiven Markierung bei einer Temperatur unter 50°C, vorzugsweise bei Umgebungs- oder Raumtemperatur (z. B. zwischen 20 und 30°C), durchgeführt wird.

14. Verfahren zur Herstellung eines Kits zur radioaktiven Markierung nach einem der Ansprüche 1 bis 8, umfassend die folgenden Schritte:
a1) Herstellen oder Bereitstellen einer Lösung, die eine geeignete Menge Acetatsalz oder -puffer zum Ausgleichen wenigstens des Saurer-pH-Eluats aus einem Gallium-68-Generator auf einen pH-Wert im Bereich von 3 bis 5 umfasst,
a2) Herstellen oder Bereitstellen eines mit einer Chelatfunktion versehenen Targeting-Mittels und
a3) Herstellen oder Bereitstellen des Metallhemmers; und
b) Lyophilisieren der in Schritt a1) bis a3) erhaltenen Lösung.

15. Verfahren zur Herstellung eines Kits zur radioaktiven Markierung nach einem der Ansprüche 1 bis 8, umfassend die folgenden Schritte:
a) Herstellen oder Bereitstellen einer Lösung, die ein mit einer Chelatfunktion versehenes Targeting-Mittel und den Metallhemmer umfasst;
b) Lyophilisieren der in Schritt a) erhaltenen Lösung und
c1) Zugeben des Acetatsalzes als Pulver im erhaltenen lyophilisierten Produkt in Schritt b) oder
c2) Zugeben eines Acetatpuffers im erhaltenen lyophilisierten Produkt in Schritt b); oder
umfassend die folgenden Schritte:
a) Herstellen einer Lösung, die das Acetatsalz, ein mit einer Chelatfunktion versehenes Targeting-Mittel und den Metallhemmer umfasst; und
b) gegebenenfalls Einfrieren der in Schritt a) erhaltenen Lösung.

16. Verfahren zur radioaktiven Markierung eines mit einer Chelatfunktion versehenes Targeting-Mittels mit Gallium-68, wobei ein Metallhemmer entweder im Eluat des Gallium-68-Generators oder in der vor Elution des Gallium-68-Generators zugegebenen HCl-Lösung enthalten ist, wobei der Metallhemmer in der Lösung zur radioaktiven Markierung vorliegt,
wobei der Metallhemmer ausgewählt ist aus der Gruppe umfassend: DOTA, Monosaccharide und Disaccharide, wobei es sich bei dem Targeting-Mittel um ein Peptid handelt, das im Wesentlichen aus 2 bis 20 Aminosäuren besteht, und
wobei die Chelatfunktionsgruppe des Targeting-Mittels ausgewählt ist aus der Gruppe umfassend: NOTA, Tris(hydroxypyridinon) (THP), offenkettige Chelatbildner wie HBED, DFO, EDTA, 6SS, B6SS, PLED, TAME und YM103; NTP(PRHP)₃, H₂dedpa, (4,6-MeO₂sal)₂-BAPEN und Citrat,
wobei der Metallhemmer und das mit einer Chelatfunktion versehene Targeting-Mittel nicht chemisch verknüpft sind.

## Revendications

1. Kit de radiomarquage comprenant :
- une quantité appropriée de sel ou tampon acétate pour équilibrer au moins l'éluat à pH acide provenant d'un générateur de gallium-68 à une valeur de pH allant de 3 à 5 lorsque ledit générateur est élué dans le kit ;
- un agent de ciblage fonctionnalisé par un chélate, ladite fonction chélate pouvant chélater du gallium-68 dans les conditions de radiomarquage ; et
- un inhibiteur de métaux, qui est un agent co-chélatant, capable d'inactiver des métaux contaminants sans interférer avec la chélation entre le gallium-68 et ledit agent de ciblage fonctionnalisé par un chélate, dans les conditions de la réaction de marquage,
dans lequel ladite réaction de radiomarquage est mise en œuvre à une température proche de la température ambiante ou égale à celle-ci et à un pH compris entre 3 et 5, dans lequel ledit inhibiteur de métaux est choisi dans le groupe comprenant : le DOTA, les monosaccharides et les disaccharides,
dans lequel ledit agent de ciblage est un peptide constitué essentiellement de 2 à 20 acides aminés et dans lequel ledit groupe fonctionnel chélate de l'agent de ciblage est choisi dans le groupe comprenant : le NOTA, la tris(hydroxypyridinone) (THP), les chélateurs à chaîne ouverte tels que le HBED, la DFO, l'EDTA, le 6SS, le B6SS, le PLED, le TAME et le YM103 ; le NTP(PRHP)₃, la H₂dedpa, le (4,6-MeO₂sal)₂-BAPEN et le citrate,
dans lequel ledit inhibiteur de métaux et ledit agent de ciblage fonctionnalisé par un chélate ne sont pas chimiquement liés.

2. Kit selon la revendication 1, dans lequel lesdits sel acétate, agent de ciblage fonctionnalisé par un chélate et inhibiteur de métaux sont (co-)lyophilisés.

3. Kit selon la revendication 1, dans lequel lesdits agent de ciblage fonctionnalisé par un chélate et inhibiteur de métaux sont (co-)lyophilisés et dans lequel ledit sel ou tampon acétate est présent séparément et peut être ajouté par la suite, ou
dans lequel lesdits agent de ciblage fonctionnalisé par un chélate et inhibiteur de métaux sont (co-)lyophilisés et du tampon acétate étant ajouté par la suite.

4. Kit selon l'une quelconque des revendications 1 à 3, dans lequel le sel acétate ou tampon au sel acétate comprend une quantité de sel acétate adaptée au type du générateur de gallium-68 utilisé.

5. Kit selon l'une quelconque des revendications 1 à 3, dans lequel le sel acétate ou tampon au sel acétate est présent en une concentration fixe pour équilibrer l'éluat à pH acide provenant d'un générateur de gallium-68 à une valeur de pH allant de 3 à 5 lorsque ledit générateur est élué dans le kit après l'ajout d'HCl au kit.

6. Kit selon l'une quelconque des revendications 1 à 5, dans lequel le groupe fonctionnel chélate de l'agent de ciblage est capable de former un complexe stable avec Ga³⁺.

7. Kit selon l'une quelconque des revendications 1 à 6, dans lequel le sel acétate est présent en une quantité comprise entre 5 mg et 1000 mg, de préférence en une quantité comprise entre 10 mg et 750 mg, de préférence encore en une quantité comprise entre 20 mg et 500 mg.

8. Kit selon l'une quelconque des revendications 1 à 7, dans lequel l'inhibiteur de métaux est présent en une quantité micromolaire, de préférence en une quantité nanomolaire, de préférence encore en une quantité au-dessous de 500 nanomoles, de préférence même encore en une quantité au-dessous de 100 nanomoles.

9. Utilisation du kit selon l'une quelconque des revendications 1 à 8, pour le radiomarquage d'un agent de ciblage fonctionnalisé par un chélate avec du gallium-68 mis en œuvre à une température proche de la température ambiante ou égale à celle-ci.

10. Procédé pour le radiomarquage d'un agent de ciblage fonctionnalisé par un chélate avec du gallium-68, comprenant l'élution d'un générateur de gallium-68 avec un éluant comprenant un acide, dans un kit selon l'une quelconque des revendications 1 à 8, de préférence dans lequel l'acide est HCl.

11. Procédé selon la revendication 10, comprenant de plus l'étape consistant à ajouter de l'HCl au kit avant l'élution.

12. Procédé selon la revendication 10 ou 11, dans lequel le radiomarquage est effectué à un pH compris entre 3 et 5, de préférence entre 3,5 et 4,5, de préférence encore entre 3,9 et 4,3.

13. Procédé selon l'une quelconque des revendications 10 à 12, dans lequel la réaction de radiomarquage est mise en œuvre à une température au-dessous de 50 °C, de préférence à température du laboratoire ou ambiante (par exemple comprise entre 20 et 30 °C).

14. Procédé pour la préparation d'un kit de radiomarquage selon l'une quelconque des revendications 1 à 8, comprenant les étapes consistant à :
a1) préparer ou fournir une solution comprenant une quantité appropriée de sel ou tampon acétate pour équilibrer au moins l'éluat à pH acide provenant d'un générateur de gallium-68 à une valeur de pH allant de 3 à 5,
a2) préparer ou fournir un agent de ciblage fonctionnalisé par un chélate et
a3) préparer ou fournir l'inhibiteur de métaux ; et
b) lyophiliser la solution obtenue dans les étapes a1) à a3).

15. Procédé pour la préparation d'un kit de radiomarquage selon l'une quelconque des revendications 1 à 8, comprenant les étapes consistant à :
a) préparer ou fournir une solution comprenant un agent de ciblage fonctionnalisé par un chélate et l'inhibiteur de métaux ;
b) lyophiliser la solution obtenue dans l'étape a) et
c1) ajouter le sel acétate sous forme d'une poudre dans le produit lyophilisé obtenu dans l'étape b), ou
c2) ajouter un tampon acétate dans le produit lyophilisé obtenu dans l'étape b) ; ou
comprenant les étapes consistant à :
a) préparer une solution comprenant le sel acétate, un agent de ciblage fonctionnalisé par un chélate et l'inhibiteur de métaux ; et
b) éventuellement congeler la solution obtenue dans l'étape a).

16. Procédé pour le radiomarquage d'un agent de ciblage fonctionnalisé par un chélate avec du gallium-68, dans lequel un inhibiteur de métaux est inclus soit dans l'éluat du générateur de gallium-68, soit dans la solution d'HCl ajoutée avant l'élution du générateur de gallium-68, dans lequel ledit inhibiteur de métaux est présent dans la solution de radiomarquage,
dans lequel ledit inhibiteur de métaux est choisi dans le groupe comprenant : le DOTA, les monosaccharides et les disaccharides,
dans lequel ledit agent de ciblage est un peptide constitué essentiellement de 2 à 20 acides aminés et dans lequel ledit groupe fonctionnel chélate de l'agent de ciblage est choisi dans le groupe comprenant : le NOTA, la tris(hydroxypyridinone) (THP), les chélateurs à chaîne ouverte tels que le HBED, la DFO, l'EDTA, le 6SS, le B6SS, le PLED, le TAME et le YM103 ; le NTP(PRHP)₃, la H₂dedpa, le (4,6-MeO₂sal)₂-BAPEN et le citrate,
dans lequel ledit inhibiteur de métaux et ledit agent de ciblage fonctionnalisé par un chélate ne sont pas chimiquement liés.
